(19) Europäisches Patentamt / European Patent Office / Office européen des brevets

(11) **EP 1 045 246 B1**

(12) **FASCICULE DE BREVET EUROPEEN**

(45) Date de publication et mention
de la délivrance du brevet:
**23.04.2008 Bulletin 2008/17**

(51) Int Cl.:
**G01N 33/22** *(2006.01)*

(21) Numéro de dépôt: **00400653.2**

(22) Date de dépôt: **09.03.2000**

(54) **Evaluation et régulation de la puissance thermique d'un flux de gaz combustible; caractérisation d'un débitmètre massique thermique**

Bestimmung und Regelung der thermischen Leistung eines Stromes von brennbarem Gas, sowie Karakterisierung eines thermischen Massenflussmessers

Evaluation and regulation of the thermal power of a stream of combustible gas, and characterization of thermal mass flowmeter

(84) Etats contractants désignés:
**AT BE CH CY DE DK ES FI FR GB GR IE IT LI LU MC NL PT SE**

(30) Priorité: **15.04.1999 FR 9904748**

(43) Date de publication de la demande:
**18.10.2000 Bulletin 2000/42**

(73) Titulaire: **L'AIR LIQUIDE, Société Anonyme pour l'Etude et l'Exploitation des Procédés Georges Claude 75007 Paris (FR)**

(72) Inventeur: **Philippe, Louis**
**78117 Toussus le Noble (FR)**

(74) Mandataire: **Vesin, Jacques et al**
**L'AIR LIQUIDE, SOCIETE ANONYME POUR L'ETUDE ET L'EXPLOITATION DES PROCEDES GEORGES CLAUDE 75, quai d'Orsay 75321 Paris Cédex 07 (FR)**

(56) Documents cités:
EP-A- 0 323 658          EP-A- 0 560 501
GB-A- 2 296 091

**Description**

La présente invention concerne:

- un procédé et un dispositif d'évaluation de la puissance thermique (ou calorifique) d'un flux d'un gaz combustible,
- un procédé et un dispositif de régulation de la puissance thermique (ou calorifique) d'un flux d'un gaz combustible,
- une méthode de caractérisation d'un débitmètre de technologie massique thermique.

Les débitmètres massiques à fil chaud sont bien connus pour des usages industriels ou de laboratoire. Leur propriété d'offrir une grande plage de mesure les rend particulièrement attractifs : on peut les utiliser dans des plages de mesure variant de 1 à 100. Ces débitmètres indiquent, de façon connue en soi, des débits massiques ou volumiques, dans des conditions normales de température et de pression. En effet, leur principe de fonctionnement est basé sur la mesure d'une différence de température ($\Delta\theta$), proportionnelle au produit de la capacité calorifique (Cp) du gaz, de la masse volumique ($\rho$) dudit gaz et du débit volumique ($\phi_v$) dudit gaz, dans les conditions normales de température et de pression :

$$\Delta\theta = k\, Cp\, \rho\, \phi_v$$

$$= k\, Cp\, \phi_m \; (\phi_m : \text{débit massique dudit gaz}).$$

L'utilisation de débitmètres massiques à fil chaud pour la mesure de débits volumiques de gaz nécessite de connaître, entre autres, la masse volumique p du gaz et la capacité calorifique Cp du gaz considéré. Avant son utilisation, un débitmètre massique doit donc être étalonné pour le gaz dont il va mesurer les débits. Pour mesurer le débit d'un deuxième gaz, il y a lieu de multiplier le débit indiqué par le débitmètre massique par un facteur correctif F. Si la composition du deuxième gaz est proche de celle du gaz pour lequel le débitmètre a été étalonné, le facteur de correction F peut être approximé par $\rho_1 Cp_1$ divisé par $\rho_2 Cp_2$, l'indice 1 correspondant au gaz pour lequel le débitmètre a été étalonné et l'indice 2 correspondant au deuxième gaz pour lequel on mesure le débit. Ainsi pour qu'un débitmètre délivre une valeur exacte de débit d'un gaz donné, il faut soit l'étalonner avec ce gaz, soit le calibrer en appliquant à la mesure un facteur correctif.

Pour le gaz naturel, ce problème est difficile à résoudre puisque la composition chimique du gaz varie en fonction de la localisation du point d'utilisation et de la source d'approvisionnement ; ladite source d'approvisionnement étant susceptible de varier dans le temps. Les imprécisions sur la mesure de débit volumique peuvent atteindre plus de 10 % pour un débitmètre étalonné pour du méthane pur, utilisé avec un gaz naturel.

Pour un industriel utilisant un tel gaz à titre de gaz combustible, les variations de la composition chimique dudit gaz sont sources de problèmes, puisque les changements de composition chimique se traduisent par des changements de pouvoir calorifique. Il est donc difficile d'évaluer et de réguler, avec une bonne précision, une puissance thermique (produit du débit volumique mesuré dans des conditions normales de température et de pression par le pouvoir calorifique (inférieur ou supérieur)) d'un flux de gaz à partir de la connaissance d'un débit donné par un débitmètre massique.

L'objet de l'invention est de résoudre cette difficulté en proposant des procédés et des dispositifs judicieux permettant respectivement d'évaluer et de réguler la puissance thermique d'un flux d'un gaz combustible.

Plus précisément, l'invention propose un procédé d'évaluation de la puissance thermique inférieure (ou supérieure) P d'un flux d'un gaz combustible de composition proche de celle d'un gaz de référence (par conséquent, lui-même combustible), ledit flux de gaz passant par un débitmètre de technologie massique thermique étalonné, voire étalonné et calibré, pour ledit gaz de référence, comportant :

- une étape de recueil de l'information donnée par ledit débitmètre,
- une étape de détermination de l'indice de Wobbe du gaz, et
- une étape de calcul de ladite puissance thermique inférieure (ou supérieure) P, dans laquelle on multiplicatie l'information recueillie par ledit débimètre par le pouvoir calorifique inférieur (ou supérieur) dudit gaz de référence et par une fonction essentiellement affine en l'indice de Wobbe du gaz.

L'homme du métier aura compris qu'il existe deux contextes de mise en oeuvre du procédé de l'invention précisé ci-dessus :

- contexte a : le débitmètre utilisé a été étalonné pour le gaz de référence (gaz étalon = ledit gaz de référence), alors, la mesure lue sur ledit débitmètre, lors du passage dudit gaz de référence correspond au débit réel ;

- contexte b : le débitmètre utilisé a été étalonné pour un gaz différent du gaz de référence (gaz étalon ≠ dudit gaz de référence), alors, la mesure lue sur ledit débitmètre, lors du passage dudit gaz de référence, doit être corrigée pour correspondre au débit réel de gaz de référence. Le facteur de correction ($F_{réf/étal}$) peut être approximé par le

rapport des $\rho Cp$ $\left( F_{réf/étal} = \dfrac{\rho_{étal}\ Cp_{étal}}{\rho_{réf}\ Cp_{réf}} \right)$ ;

A l'issue de ces étapes d'étalonnage, voire d'étalonnage et de calibrage, le débitmètre intervenant donne une mesure de débit exacte lorsqu'il est traversé par le gaz de référence.

L'invention propose également un procédé de régulation à une valeur $P_o$, de la puissance thermique inférieure (ou supérieure) P d'un flux d'un gaz combustible de composition proche de celle d'un gaz de référence (par conséquent, lui-même combustible), ledit flux de gaz passant par un débitmètre de technologie massique thermique étalonné, voire étalonné et calibré, pour ledit gaz de référence, comportant :

- l'évaluation de ladite puissance thermique inférieure (ou supérieure) P, selon le procédé précisé ci-dessus,
- la régulation de ladite puissance P (selon des méthodes de régulation connues en elles-mêmes) à ladite valeur $P_0$.

La régulation ci-dessus peut évidemment également être mise en oeuvre dans les deux contextes a et b précisés ci-dessus.

Sa mise en oeuvre peut se décliner selon plusieurs variantes. On peut notamment réguler le débit de gaz combustible intervenant ou la composition dudit gaz.

Les procédés ci-dessus reposent sur la découverte par l'inventeur d'une corrélation simple entre l'indice de Wobbe (W) et le ratio entre la puissance thermique inférieure réelle à un brûleur (P) et la puissance apparente (Pc), calculée par le produit du débit volumique apparent donné par un débitmètre massique étalonné, voire étalonné et calibré, pour le gaz de référence par le pouvoir calorifique inférieur dudit gaz de référence ; corrélation affine qui s'écrit :

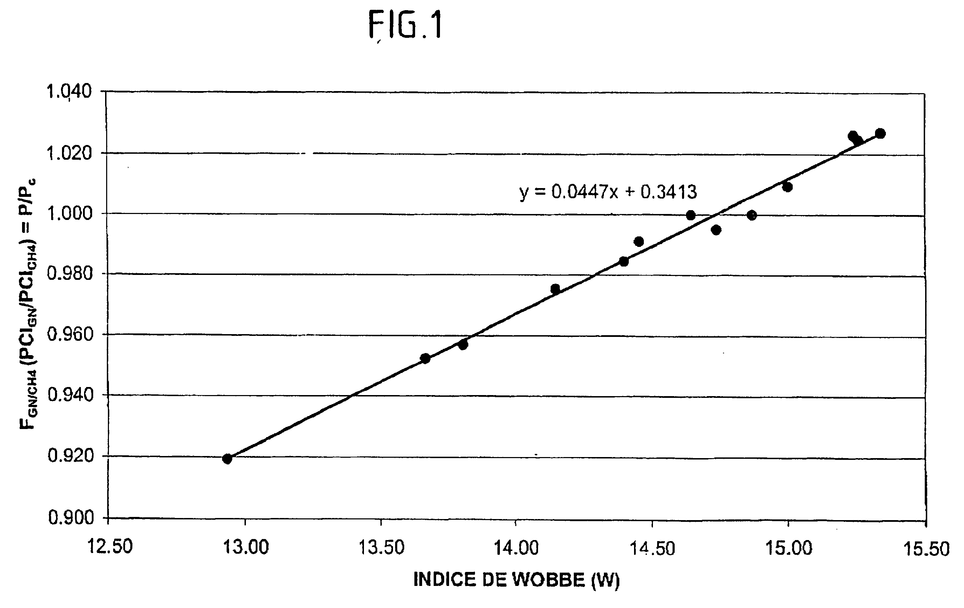

$$\frac{P}{Pc} = f(W) = a\,W + b \text{ (voir la figure 1 annexée).}$$

On parle présentement de fonction "essentiellement" affine dans la mesure où il n'est pas exclu du tout, du cadre de l'invention, de peaufiner la corrélation ci-dessus, d'y adjoindre au moins un terme quadratique. Une précision plus que satisfaisante est en tout état de cause obtenue avec une fonction du premier degré.

Les coefficients qui interviennent dans ladite corrélation (coefficients caractérisant le débitmètre) dépendent évidemment de la nature du gaz combustible de référence.

Dans la mesure où il intervient généralement pour la mise en oeuvre de la combustion du gaz, un gaz comburant, les procédés d'évaluation et de régulation tels que définis ci-dessus comportent avantageusement aussi une étape de régulation du débit de comburant intervenant à la valeur $Q_0$' égale à (1+x) P/K où x est l'excès de comburant (x s'exprimant en pourcentage, par rapport au débit de comburant nécessaire à la combustion stoechiométrique du gaz) et où K est une constante dépendant du comburant.

On a compris que, lorsque l'on s'intéresse à la puissance thermique inférieure du gaz, on fait intervenir le pouvoir calorifique inférieur du gaz de référence et que, lorsque l'on s'intéresse à la puissance supérieure dudit gaz, on fait intervenir le pouvoir calorifique supérieur dudit gaz de référence.

Avantageusement, ledit gaz de référence est :

- du méthane pur ou un gaz naturel de référence, lorsque le gaz combustible en question est un gaz naturel ;
- du propane pur ou un propane de référence, lorsque le gaz combustible en question est du propane industriel ;
- du butane pur ou un butane de référence, lorsque le gaz combustible en question est du butane industriel.

Dans le cadre d'une variante avantageuse de mise en oeuvre des procédés de l'invention, le gaz combustible intervenant est un gaz naturel, le gaz de référence est du méthane et la fonction essentiellement affine s'écrit :

$$f(W) = a\,W + b,$$

a étant compris entre 0,035 et 0,050 et b étant compris entre 0,30 et 0,45. Les coefficients a et b peuvent en effet varier quelque peu en fonction de la mise en oeuvre de la méthode de caractérisation du débitmètre telle que décrite plus loin.

Pour la détermination de l'indice de Wobbe du gaz intervenant, on peut opérer de diverses manières. On peut notamment placer sur le réseau d'alimentation en ledit gaz, "un analyseur" qui mesure des quantités physiques dudit gaz (telles son pouvoir calorifique supérieur, sa densité par rapport à l'air, sa composition chimique, son indice de comburité ...) à partir desquelles l'indice de Wobbe est calculé. Un tel analyseur peut consister en un comburimètre qui mesure l'indice de comburité du gaz, indice de comburité directement proportionnel à l'indice de Wobbe. Il peut également consister en un chromatographe qui détermine la composition chimique du gaz, composition chimique à partir de laquelle l'indice de Wobbe est calculé. Il a avantageusement un temps de réponse rapide. Il n'est nullement exclu de mesurer directement ledit indice de Wobbe par des méthodes calorimétriques.

De préférence, pour la mise en oeuvre des procédés de l'invention, on utilise un comburimètre pour la détermination de l'indice de Wobbe du gaz.

On rappelle à toutes fins utiles que ledit indice de Wobbe est défini par le rapport entre le pouvoir calorifique supérieur du gaz et la racine carrée de la densité dudit gaz.

On a compris ici que, pour la mise en oeuvre des procédés de l'invention décrits ci-dessus, un travail préalable a été accompli sur le débitmètre utilisé, en référence à une famille donnée de gaz combustible, pour laquelle il va être utilisé. On peut parler d'une étape préalable de caractérisation dudit débitmètre, de technologie massique thermique. Une telle méthode de caractérisation d'un tel débitmètre constitue un autre objet de la présente invention. Elle est décrite ci-dessous.

Ladite méthode de caractérisation d'un débitmètre de technologie massique thermique - destiné à mesurer des débits volumiques d'un gaz combustible GC d'une famille donnée, ledit débitmètre ayant été préalablement étalonné, voire étalonné et calibré, avec un gaz de référence GCo (ledit gaz de référence GCo présentant une composition proche de celle dudit gaz combustible GC) - consiste à adjoindre audit débitmètre deux coefficients a et b, calculés comme les coefficients de la loi linéaire qui approche la relation existant entre le produit $F_{GC/GCo}$ ($PC_{GC}/PC_{GCo}$) et l'indice de Wobbe $W_{GC}$ du gaz combustible GC dont on vise à mesurer les débits volumiques; loi linéaire qui s'énonce :

$$F_{GC/GCo} \, (PC_{GC}/PC_{GCo}) = a \, W_{GC} + b \, (= \frac{P_{GC}}{P_{GCo}} \text{ où } P_{GC} \text{ est la puissance réelle et } P_{GCo} \text{ est la}$$

puissance apparente, puissances qui se calculent par le produit des débits par les pouvoirs calorifiques)

avec :

$F_{GC/GCo}$: facteur de correction à appliquer à la mesure du débit volumique donnée par le débitmètre pour connaître le débit réel volumique de gaz combustible GC ;

$PC_{GC}/PC_{GCo}$ : rapport du pouvoir calorifique (inférieur ou supérieur) du gaz combustible Gc dont on vise à mesurer le débit volumique sur le pouvoir calorifique (inférieur ou supérieur) correspondant du gaz combustible de référence GCo.

Pour la mesure du débit volumique, ledit facteur de correction $F_{GC/GCo}$ peut être approximé par le rapport :

$$\frac{\rho_{GCo} \, Cp_{GCo}}{\rho_{GC} \, Cp_{GC}} \quad ;$$

avec :

$\rho$ : masse volumique du gaz considéré
$Cp$ : capacité calorifique du gaz considéré.

Il n'est pas exclu de calculer ledit facteur de correction par une autre méthode.

Les pouvoirs calorifiques $PC_{GC}$ et $PC_{GCo}$, sont, tous deux, soit des pouvoirs calorifiques inférieurs, soit des pouvoirs calorifiques supérieurs.

Pour ce qui concerne lesdits coefficients a et b de la fonction affine appelée à caractériser le débitmètre utilisé, ils sont avantageusement calculés de la manière suivante :

a) on sélectionne un nombre significatif, au sens statistique, de compositions représentatives de gaz combustible : GC, $GC_1$, $GC_2$, ... $GC_n$ ;
b) pour chacune desdites n compositions, on calcule ou mesure :

- l'indice de Wobbe : $W_{GC1}$ ;
- un même pouvoir calorifique : $PC_{GCi}$ ; par exemple le pouvoir calorifique inférieur $PCI_{GCi}$ ou le pouvoir calorifique supérieur $PCS_{GCi}$ ;
- le facteur de correction : $F_{GCi/GCo}$ ;

c) pour chacune desdites n compositions, on calcule alors le produit : $X_{GCi} = F_{GCi/GCo} (PC_{GCi}/PC_{GCo})$;
d) on calcule enfin a et b par une régression linéaire entre les n couples de données : $(X_{GC1}, W_{GC1})$.

Dans ledit calcul, lesdits facteurs de correction peuvent être approximés par le rapport :

$$\frac{\rho_{GCo}\ Cp_{GCo}}{\rho_{GCi}\ Cp_{GCi}}$$

avec :

$\rho$ : masse volumique du gaz considéré
Cp : capacité calorifique du gaz considéré.

L'invention propose également un dispositif d'évaluation de la puissance thermique inférieure (ou supérieure) P d'un flux d'un gaz combustible de composition proche de celle d'un gaz de référence, comportant :

- un débitmètre de technologie massique thermique étalonné pour ledit gaz de référence,
- un moyen de détermination de l'indice de Wobbe du gaz,
- un moyen de recueil de l'information donnée par ledit débitmètre, et dudit indice de Wobbe, et,
- un moyen de calcul de ladite puissance thermique inférieure (ou supérieure) P arrangé pour effectuer la multiplication de l'information recueillie par ledit débitmètre par le pouvoir calorifique inférieur (ou supérieur) dudit gaz de référence et par une fonction essentiellement affine en l'indice de Wobbe du gaz.

L'invention propose aussi un dispositif de régulation à une valeur $P_0$, de la puissance thermique inférieure (ou supérieure) P d'un flux d'un gaz combustible de composition proche de celle d'un gaz de référence, comportant :

- le dispositif d'évaluation de ladite puissance thermique inférieure (ou supérieure) P, tel que défini ci-dessus, et
- un moyen de régulation (moyen connu en soi) de ladite puissance P à ladite valeur $P_0$.

Ledit moyen de régulation peut intervenir soit sur le débit du gaz, soit sur la composition dudit gaz (*ie*. sur son indice de Wobbe), voire conjointement sur ces deux paramètres. Il est donc susceptible d'inclure au moins une vanne de régulation de débit ou/et au moins un moyen de mélange d'un autre gaz dans ledit gaz.
Avantageusement, le dispositif de régulation comporte également un moyen de régulation du débit de comburant intervenant à la valeur $Q_0$' égale à $(1+x)$ P/K où x est l'excès de comburant et où K est une constante dépendant du comburant.
De préférence, le dispositif comporte un comburimètre pour la détermination de l'indice de Wobbe du gaz. Ledit comburimètre peut être disposé en amont ou en aval du débitmètre par rapport au sens du flux de gaz.
Selon un autre mode de réalisation, le moyen de détermination de l'indice de Wobbe du gaz comporte un analyseur de la composition chimique dudit gaz.
L'invention décrite ci-dessus repose sur l'utilisation de l'indice de Wobbe (W) comme grandeur caractéristique du gaz combustible (GC). Il est bien évident que les procédés et dispositifs décrits pourraient tout aussi bien être basés sur l'utilisation d'un indice équivalent, tel l'indice de comburité B. L'homme du métier sait que ledit indice de comburité (B) est, pour le gaz naturel, lié audit indice de Wobbe (W), par la formule : $B = \dfrac{W}{1,16}$ .

Dans la présente description et les revendications annexées, le terme "indice de Wobbe" englobe donc l'indice de Wobbe proprement dit et les indices équivalents.

D'autres caractéristiques et avantages de l'invention apparaîtront dans la description qui va suivre, d'un exemple, décrit en référence aux figures annexées, sur lesquelles :

- la figure 1 représente, pour treize compositions de gaz naturels et en fonction de l'indice de Wobbe desdits gaz naturels, le produit :

$$F_{GN/CH_4} \, (PCI_{GN} \, /PCI_{CH_4})$$

avec $F_{GN/CH_4}$, facteur de correction, approximé par la formule

$$\frac{\rho_{CH_4} \; Cp_{CH_4}}{\rho_{GC} \; Cp_{GC}} \; ;$$

et $PCI_{GN}$, pouvoirs calorifiques inférieurs, calculés à partir de la composition des treize gaz naturels intervenants ;

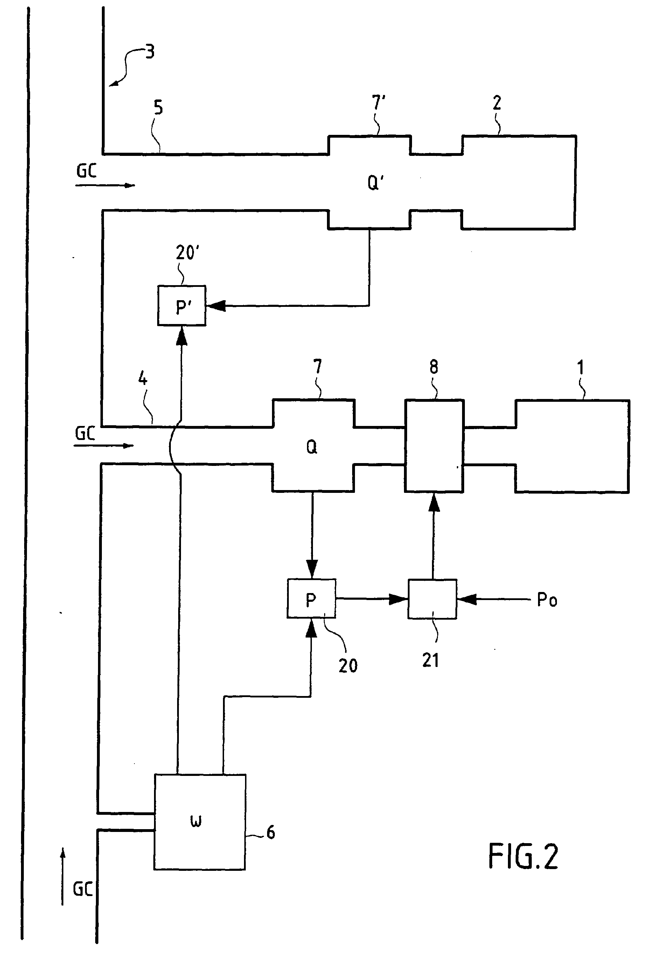

- la figure 2 représente, de façon schématique, un réseau de distribution, d'un gaz combustible GC, équipé de moyens permettant la mise en oeuvre des procédés de l'invention.

En se référant plus particulièrement à la figure 1, l'inventeur a découvert pour les gaz naturels une corrélation surprenante entre l'indice de Wobbe d'un gaz naturel, grandeur mesurable par exemple avec un comburimètre, et le rapport entre, d'une part, la puissance réelle à un brûleur notée P d'un flux dudit gaz naturel et, d'autre part, la puissance apparente notée Pc calculée par le produit du pouvoir calorifique inférieur du méthane, noté $PCI_{CH_4}$, par le débit volumique, noté $Q_{CH_4}$, du flux dudit gaz naturel, indiqué par un débitmètre massique étalonné pour du méthane.

Cette relation s'écrit, en posant $Pc = PCI_{CH_4} \, Q_{CH_4}$, et en notant "W" l'indice de Wobbe :

$$P \, / \, Pc = a \, W + b$$

avec $a = 4,47 \; 10^{-2}$
et $b = 0,3413$.

Cette relation est indépendante du gaz naturel utilisé. Sa précision est de l'ordre de 0,5 %.

Cette corrélation a été établie pour 13 compositions de gaz, des gaz disponibles en France et leurs mélanges.

Ce résultat inattendu permet d'utiliser la technologie de débitmètre massique à fil chaud pour la régulation de la puissance d'une installation de combustion, et pour la régulation du rapport de combustion, à l'air ou à l'oxygène, indépendamment de la composition du gaz naturel.

On propose les commentaires ci-après en référence à la figure 2.

Le réseau de distribution schématisé alimente deux équipements de combustion 1 et 2 (du type brûleurs, fours, par exemple) *via* la conduite principale 3 et les conduites secondaires 4 et 5.

Ladite conduite principale 3 est équipé, en amont desdites conduites secondaires 4 et 5, d'un appareil 6 qui permet de calculer l'indice de Wobbe W du gaz GC. La valeur dudit indice de Wobbe est transmise aux deux calculateurs 20 et 20'.

Sur la conduite secondaire 5, il est monté un débitmètre 7' de technologie massique thermique, étalonné pour un gaz de référence, de composition proche de celle dudit gaz GC.

L'invention est mise en oeuvre dans la mesure où la composition dudit gaz G est susceptible de varier au cours du temps (tout en restant proche de celle dudit gaz de référence).

La puissance thermique inférieure (ou supérieure) P' avec laquelle est alimenté l'équipement de combustion 2 est calculée, selon l'invention, dans le calculateur 20', à partir :

- de l'indice de Wobbe W, déterminé dans l'appareil 6,

- de la valeur du débit Q', lue sur le débitmètre 7',
- du pouvoir calorifique inférieur (ou supérieur) du gaz de référence (une constante),
- et de la fameuse fonction affine qui caractérise le débitmètre 7' intervenant ; selon la formule :

$$P' = PCI_{réf} \, Q'_{lu} \, ((a' \, W + b')$$

Sur la conduite secondaire 4, il est monté, en série : un débitmètre 7 de technologie massique thermique, étalonné lui-aussi pour un gaz de référence et caractérisé lui aussi par des coefficients a et b d'une fonction affine ; et un organe 8 susceptible de régler le débit d'alimentation en le gaz GC de l'équipement de combustion 1.
En effet, pour l'alimentation dudit équipement de combustion 1, il est prévu une régulation de la puissance thermique inférieure (ou supérieure) P.
Ladite puissance thermique inférieure (ou supérieure) P est calculée, selon l'invention, dans le calculateur 20, à partir :

- de l'indice de Wobbe W, déterminé dans l'appareil 6,
- de la valeur du débit Q, lue sur le débitmètre 7,
- du pouvoir calorifique inférieur (ou supérieur) du gaz de référence,
- et de la fonction affine qui caractérise ledit débitmètre 7, selon la formule :

$$P = PCI_{ref} \, Q_{lu} \, (a \, W + b).$$

Ladite puissance thermique inférieure (ou supérieure) P est régulée à la valeur $P_0$ *via* le régulateur 21.
Ledit régulateur 21 commande l'organe 8 de réglage du débit.

## Revendications

1.  Procédé d'évaluation de la puissance thermique inférieure, ou supérieure, P, d'un flux d'un gaz combustible GC de composition proche de celle d'un gaz de référence, ledit flux de gaz passant par un débitmètre (7) de technologie massique thermique étalonné, voire étalonné et calibré, pour ledit gaz de référence, comportant :

    - une étape de recueil de l'information donnée par ledit débitmètre (7),
    - une étape de détermination de l'indice de Wobbe du gaz, et
    - une étape de calcul de ladite puissance thermique inférieure, ou supérieure, P, dans laquelle on multiplicatie l'information recueillie par ledit débimètre par le pouvoir calorifique inférieur, ou supérieur, dudit gaz de référence et par une fonction essentiellement affine en l'indice de Wobbe du gaz.

2.  Procédé de régulation à une valeur $P_0$, de la puissance thermique inférieure, ou supérieure, P d'un flux d'un gaz combustible GC de composition proche de celle d'un gaz de référence, ledit flux de gaz passant par un débitmètre (7) de technologie massique thermique étalonné, voire étalonné et calibré, pour ledit gaz de référence, comportant :

    - l'évaluation de ladite puissance thermique inférieure, ou supérieure, P, selon le procédé de la revendication 1, et
    - la régulation de ladite puissance à ladite valeur $P_0$.

3.  Procédé selon l'une des revendications 1 ou 2, **caractérisé en ce qu'**il comporte en outre une étape de régulation du débit de comburant intervenant à la valeur $Q_0'$ égale à (1+x) P/K où x est l'excès de comburant et où K est une constante dépendant du comburant.

4.  Procédé selon l'une quelconque des revendications 1 à 3, **caractérisé en ce que** le gaz de référence est du méthane, du propane ou du butane.

5.  Procédé selon l'une quelconque des revendications 1 à 4, **caractérisé en ce que**, le gaz combustible GC intervenant étant un gaz naturel, le gaz de référence étant du méthane, la fonction essentiellement affine s'écrit :

$$f(W) = a\ W + b,$$

a étant compris entre 0,035 et 0,050 et b étant compris entre 0,30 et 0,45.

6. Procédé selon l'une quelconque des revendications 1 à 5, **caractérisé en ce qu'**on utilise un comburimètre pour la détermination de l'indice de Wobbe du gaz.

7. Procédé selon l'une quelconque des revendications 1 à 5, **caractérisé en ce que** la détermination de l'indice de Wobbe du gaz est effectuée par un calcul à partir des résultats de l'analyse chimique dudit gaz.

8. Dispositif d'évaluation de la puissance thermique inférieure, ou supérieure, P, d'un flux d'un gaz combustible GC de composition proche de celle d'un gaz de référence, comportant :

   - un débitmètre (7) de technologie massique thermique étalonné pour ledit gaz de référence,
   - un moyen de détermination (6) de l'indice de Wobbe du gaz,
   - un moyen de recueil (20) de l'information donnée par ledit débitmètre (7), et dudit indice de Wobbe,

   et un moyen de calcul (20) de ladite puissance thermique inférieure, ou supérieure, P, arrangé pour effectuer la multiplication de l'information recueillie par ledit débitmètre (7) par le pouvoir calorifique inférieur, ou supérieur, dudit gaz de référence et par une fonction essentiellement affine en l'indice de Wobbe du gaz.

9. Dispositif de régulation à une valeur $P_0$, de la puissance thermique inférieure, ou supérieure, P d'un flux d'un gaz combustible GC de composition proche de celle d'un gaz de référence, comportant :

   - un dispositif d'évaluation de ladite puissance thermique inférieure, ou supérieure, P selon la revendication 8, et
   - un moyen de régulation (8, 21, 22) de ladite puissance P à ladite valeur $P_0$

10. Dispositif selon la revendication 9, **caractérisé en ce qu'**il comporte en outre un moyen de régulation du débit de comburant intervenant à la valeur $Q_0'$ égale à (1+x) P/K où x est l'excès de comburant et où K est une constante dépendant du comburant.

11. Dispositif selon l'une quelconque des revendications 8 à 10, **caractérisé en ce** ledit moyen de détermination (6) de l'indice de Wobbe du gaz inclut un comburimètre.

12. Dispositif selon l'une quelconque des revendications 8 à 10, **caractérisé en ce que** ledit moyen de détermination de l'indice de Wobbe du gaz comporte un analyseur de la composition chimique dudit gaz.

13. Méthode de caractérisation d'un débitmètre de technologie massique thermique, destiné à mesurer des débits volumiques d'un gaz combustible GC d'une famille donnée, ledit débitmètre ayant été préalablement étalonné, voire étalonné et calibré avec un gaz de référence GCo ; ladite méthode consistant à adjoindre audit débitmètre deux coefficients a et b, calculés comme les coefficients de la loi linéaire qui approche la relation existant entre le produit $F_{GC/GCo}$ ($PC_{GC}/PC_{GCo}$) et l'indice de Wobbe $W_{GC}$ du gaz combustible GC dont on vise à mesurer les débits volumiques; loi linéaire qui s'énonce :

$$F_{GC/GCo}\ (PC_{GC}/PC_{GCo}) = a\ W_{GC} + b$$

avec :

   $F_{GC/GCo}$: facteur de correction à appliquer à la mesure du débit volumique donnée par le débitmètre pour connaître le débit réel volumique de gaz combustible GC ;
   $PC_{GC}/PC_{GCo}$ : rapport du pouvoir calorifique, inférieur ou supérieur, du gaz combustible GC dont on vise à mesurer le débit volumique sur le pouvoir calorifique, inférieur ou supérieur, correspondant du gaz combustible de référence GCo.

**14.** Méthode selon la revendication 13, **caractérisée en ce que** lesdits deux coefficients a et b sont calculés comme suit :

a) on sélectionne un nombre significatif, au sens statistique, de compositions représentatives de gaz combustible : GC, GC$_1$, GC$_2$, ... GC$_n$ ;
b) pour chacune desdites n compositions, on calcule ou mesure :

- l'indice de Wobbe : W$_{GCi}$ ;
- un même pouvoir calorifique : PC$_{GCi}$ ; par exemple le pouvoir calorifique inférieur PCI$_{GCi}$ ou le pouvoir calorifique supérieur PCS$_{GCi}$;
- le facteur de correction : F$_{GCi/GCo}$;

c) pour chacune desdites n compositions, on calcule alors le produit : X$_{GC1}$ = F$_{GCi/GCo}$ (PC$_{GCi}$/PC$_{GCo}$) ;
d) on calcule enfin a et b par une régression linéaire entre les n couples de données : (X$_{GCi}$, W$_{GCi}$).

**Claims**

**1.** Process for the evaluation of the net or gross thermal power P of a flow of a combustible gas CG of composition close to that of a reference gas, said flow of gas passing through a flowmeter (7) of thermal mass technology which is standardized, or standardized and calibrated, for said reference gas, comprising:

- a step of collecting the information given by said flowmeter (7);
- a step of determining the Wobbe index of the gas; and
- a step of calculating said net or gross thermal power P in which the information collected by said flowmeter is multiplied by the net or gross calorific value of said reference gas and by an essentially affine function of the Wobbe index of the gas.

**2.** Process for the regulation of the net or gross thermal power P of a flow of a combustible gas CG of composition close to that of a reference gas to a value P$_o$, said flow of gas passing through a flowmeter (7) of thermal mass technology which is standardized, or standardized and calibrated, for said reference gas, comprising:

- the evaluation of said net or gross thermal power P, according to the process of Claim 1; and
- the regulation of said power to said value P$_0$.

**3.** Process according to either of Claims 1 and 2, **characterized in that** it furthermore includes a step of regulating the flow rate of oxidant employed to the value Q$_0$' equal to (1+x) P/K, where x is the excess oxidant and where K is a constant which depends on the oxidant.

**4.** Process according to any one of Claims 1 to 3, **characterized in that** the reference gas is methane, propane or butane.

**5.** Process according to any one of Claims 1 to 4, **characterized in that**, with the combustible gas CG used being a natural gas and the reference gas being methane, the essentially affine function is written as:

$$f(W) = aW + b,$$

a being between 0.035 and 0.050 and b being between 0.30 and 0.45.

**6.** Process according to any one of Claims 1 to 5, **characterized in that** a combustibility analyzer is used for determining the Wobbe index of the gas.

**7.** Process according to any one of Claims 1 to 5, **characterized in that** the Wobbe index of the gas is determined by a calculation based on the results of the chemical analysis of said gas.

**8.** Device for evaluating the net or gross thermal power P of a flow of a combustible gas CG of composition close to that of a reference gas, comprising:

- a flowmeter (7) of thermal mass technology, which is standardized for said reference gas;
- a means (6) for determining the Wobbe index of the gas;
- a means (20) for collecting the information given by said flowmeter (7), and said Wobbe index; and
- a means (20) for calculating said net or gross thermal power P designed to multiply the information collected by said flowmeter (7) by the net or gross calorific value of said reference gas and by an essentially affine function of the Wobbe index of the gas.

9.  Device for regulating the net or gross thermal power P of a flow of a combustible gas CG of composition close to that of a reference gas to a value $P_0$, comprising:

    - a device for evaluating said net or gross thermal power P according to claim 8; and
    - a means (8, 21, 22) for regulating said power P to said value $P_0$.

10. Device according to Claim 9, **characterized in that** furthermore includes a means for regulating the flow rate of oxidant used to the value $Q_0$', equal to (1+x) P/K, where x is the excess oxidant and where K is a constant which depends on the oxidant.

11. Device according to any one of Claims 8 to 10, wherein said means (6) for determining the Wobbe index of the gas includes a combustibility analyzer.

12. Device according to any one of Claims 8 to 10, **characterized in that** said means for determining the Wobbe index of the gas comprises an analyzer for analyzing the chemical composition of said gas.

13. Method of characterizing a flowmeter of thermal mass technology, intended to measure volume flow rates of a combustible gas CG of a given family, said flowmeter having been standardized, or standardized and calibrated, beforehand with a reference gas CGo, said method consisting in adding to said flowmeter two coefficients a and b, these being calculated as the coefficients of the linear law which approaches the relationship existing between the product $F_{CG/CGo}(CV_{CG}/CV_{CGo})$ and the Wobbe index $W_{CG}$ of the combustible gas CG, the volume flow rates of which are intended to be measured, which linear law is expressed as:

$$F_{CG/CGo}(CV_{CG}/CV_{CGo}) = aW_{CG} + b$$

with:

$F_{CG/CGo}$: correction factor to be applied to the volume flow rate measurement given by the flowmeter in order to determine the actual volume flow rate of combustible gas CG;
$CV_{CG}/CV_{CGo}$: ratio of the net or gross calorific value of the combustible gas CG, a volume flow rate of which is intended to be measured, to the corresponding net or gross calorific value of the combustible reference gas CGo.

14. Method according to Claim 13, **characterized in that** said two coefficients a and b are calculated as follows:

    a) a statistically significant number of compositions representative of the combustible gas are selected: CG, $CG_1$, $CG_2$, ... $CG_n$;
    b) for each of said n compositions, the following are calculated or measured:

    - the Wobbe index: $W_{CGi}$;
    - the same calorific value: $CV_{CGi}$; for example, the net calorific value $NCV_{CGi}$ or the gross calorific value $CGV_{CGi}$;
    - the correction factor: $F_{CGi/CGo}$;

    c) for each of said n compositions, the following product is then calculated:

$$X_{CGi} = F_{CGi/CGo}(CV_{CGi}/CV_{CGo});$$

d) finally, a and b are calculated by a linear regression between the n pairs of data: ($X_{CGi}$, $W_{CGi}$).

**Patentansprüche**

1.  Verfahren zum Bewerten der unteren oder oberen Wärmeleistung P eines Stroms eines brennbaren Gases GC mit einer Zusammensetzung, die nahe bei jener eines Referenzgases liegt, wobei sich der Gasstrom durch einen Durchflussmesser (7) mit Massen-Wärme-Technologie bewegt, der für das Referenzgas geeicht oder geeicht und kalibriert ist, wobei das Verfahren enthält:

    - einen Schritt des Sammelns von durch den Durchflussmesser (7) gegebenen Informationen,
    - einen Schritt des Bestimmens des Wobbe-Indexes des Gases und
    - einen Schritt des Berechnens der unteren oder oberen Wärmeleistung P, bei dem die von dem Durchflussmesser gesammelten Informationen mit dem unteren oder oberen Heizwert des Referenzgases und mit einer Funktion, die in dem Wobbe-Index des Gases im Wesentlichen affin ist, multipliziert werden.

2.  Verfahren zum Regulieren der unteren oder oberen Wärmeleistung P eines Stroms eines brennbaren Gases GC mit einer Zusammensetzung, die nahe bei jener eines Referenzgases liegt, auf einen Wert $P_0$, wobei sich der Gasstrom durch einen Durchflussmesser (7) mit Massen-Wärme-Technologie bewegt, der für das Referenzgas geeicht oder geeicht und kalibriert ist, wobei das Verfahren enthält:

    - Bewerten der unteren oder oberen Wärmeleistung P gemäß dem Verfahren nach Anspruch 1 und
    - Regulieren der Leistung auf den Wert $P_0$.

3.  Verfahren nach einem der Ansprüche 1 oder 2, **dadurch gekennzeichnet, dass** es außerdem einen Schritt des Regulierens des Durchflusses des auftretenden Sauerstoffträgers auf den Wert $Q_o'$ umfasst, der gleich $(1 + x)P/K$ ist, wobei x der überschüssige Sauerstoffträger ist und wobei K eine vom Sauerstoffträger abhängende Konstante ist.

4.  Verfahren nach einem der Ansprüche 1 bis 3, **dadurch gekennzeichnet, dass** das Referenzgas Methan, Propan oder Butan ist.

5.  Verfahren nach einem der Ansprüche 1 bis 4, **dadurch gekennzeichnet, dass** das auftretende brennbare Gas GC ein Naturgas ist, das Referenzgas Methan ist und die im Wesentlichen affine Funktion lautet:

$$f(W) = aW + b,$$

    wobei a im Bereich zwischen 0,035 bis 0,050 liegt und b im Bereich zwischen 0,30 bis 0,45 liegt.

6.  Verfahren nach einem der Ansprüche 1 bis 5, **dadurch gekennzeichnet, dass** für die Bestimmung des Wobbe-Indexes des Gases ein Comburimeter (*comburimètre*) verwendet wird.

7.  Verfahren nach einem der Ansprüche 1 bis 5, **dadurch gekennzeichnet, dass** die Bestimmung des Wobbe-Indexes des Gases durch eine Berechnung anhand der Ergebnisse der chemischen Analyse des Gases ausgeführt wird.

8.  Vorrichtung zum Bewerten der unteren oder oberen Wärmeleistung P eines Stroms eines brennbaren Gases GC mit einer Zusammensetzung, die nahe bei jener eines Referenzgases liegt, mit:

    - einem Durchflussmesser (7) mit Massen-Wärme-Technologie, der für das Referenzgas geeicht ist,
    - einem Mittel (6) zum Bestimmen des Wobbe-Indexes des Gases,
    - einem Mittel (20) zum Sammeln von durch den Durchflussmesser (7) gegebenen Informationen und des Wobbe-Indexes,

    und einem Mittel (20) zum Berechnen der unteren oder oberen Wärmeleistung P, das ausgelegt ist, um die Multiplikation der von dem Durchflussmesser (7) gesammelten Informationen mit dem unteren oder oberen Heizwert des Referenzgases und mit einer in dem Wobbe-Index des Gases im Wesentlichen affinen Funktion auszuführen.

9. Vorrichtung zum Regulieren der unteren oder oberen Wärmeleistung P eines Stroms eines brennbaren Gases GC mit einer Zusammensetzung, die nahe bei jener eines Referenzgases liegt, auf einen Wert $P_0$, mit:

   - einer Vorrichtung zum Bewerten der unteren oder oberen Wärmeleistung P nach Anspruch 8 und
   - einem Mittel (8, 21, 22) zum Regulieren der Leistung P auf den Wert $P_0$.

10. Vorrichtung nach Anspruch 9, **dadurch gekennzeichnet, dass** sie außerdem ein Mittel enthält, um den Durchfluss des auftretenden Sauerstoffträgers auf den Wert $Q_o'$ zu regulieren, der gleich $(1 + x)P/K$ ist, wobei x der überschüssige Sauerstoffträger ist und wobei K eine von dem Sauerstoffträger abhängende Konstante ist.

11. Vorrichtung nach einem der Ansprüche 8 bis 10, **dadurch gekennzeichnet, dass** das Mittel (6) zum Bestimmen des Wobbe-Indexes des Gases ein Comburimeter (*comburimètre*) enthält.

12. Vorrichtung nach einem der Ansprüche 8 bis 10, **dadurch gekennzeichnet, dass** das Mittel zum Bestimmen des Wobbe-Indexes des Gases einen Analysator für die chemische Zusammensetzung des Gases enthält.

13. Verfahren zum Charakterisieren eines Durchflussmessers mit Massen-Wärme-Technologie, der dazu vorgesehen ist, Volumendurchflüsse eines brennbaren Gases GC einer gegebenen Familie zu messen, wobei der Durchflussmesser im Voraus mit einem Referenzgas GCo geeicht oder geeicht und kalibriert worden ist; wobei das Verfahren darin besteht, dem Durchflussmesser zwei Koeffizienten a und b zuzuweisen, die als die Koeffizienten jenes linearen Gesetzes berechnet werden, das die bestehende Relation zwischen dem Produkt $F_{GC/GCo}$ ($PC_{GC}/PC_{GCo}$) und dem Wobbe-Index $W_{GC}$ des brennbaren Gases GC, dessen Volumendurchflüsse gemessen werden sollen, approximiert, wobei das lineare Gesetz lautet:

$$F_{GC/GCo}(PC_{GC}/PC_{GCo}) = aW_{GC} + b$$

wobei gilt:

$F_{GC/GCo}$: Korrekturfaktor, der auf die Messung des durch den Durchflussmesser gegebenen Volumendurchflusses anzuwenden ist, um den wirklichen Volumendurchfluss des brennbaren Gases GC zu kennen;
$PC_{GC}/PC_{GCo}$: Verhältnis des unteren oder oberen Heizwerts des brennbaren Gases GC, dessen Volumendurchsatz gemessen werden soll, zu dem entsprechenden unteren oder oberen Heizwert des brennbaren Referenzgases GCo.

14. Verfahren nach Anspruch 13, **dadurch gekennzeichnet, dass** die beiden Koeffizienten a und b wie folgt berechnet werden:

   a) Wählen einer im statistischen Sinn signifikanten Anzahl repräsentativer Zusammensetzungen des brennbaren Gases: GC, $GC_1$, $GC_2$, ..., $GC_n$;
   b) für jede der n Zusammensetzungen Berechnen oder Messen:

   - des Wobbe-Indexes: $W_{GCi}$;
   - desselben Heizwerts: $PC_{GCi}$; beispielsweise des unteren Heizwerts $PCI_{GCi}$ oder des oberen Heizwerts $PCS_{GCi}$;
   - des Korrekturfaktors: $F_{GCi/GCo}$;

   c) anschließend Berechnen des Produkts: $X_{GCi} = F_{GCi/GCo}$ ($PC_{GCi}/PC_{GCo}$) für jede der n Zusammensetzungen;
   d) schließlich Berechnen von a und b durch eine lineare Regression zwischen den n Datenpaaren: ($X_{GCi}$, $W_{GCi}$).

FIG.1

$y = 0.0447x + 0.3413$

EP 1 045 246 B1

FIG.2